(19) Europäisches Patentamt
European Patent Office
Office européen des brevets

(11) Publication number: 0 221 214
A1

(12) # EUROPEAN PATENT APPLICATION

(21) Application number: 85307887.1

(51) Int. Cl.[4]: C07C 29/15 , C07C 31/20

(22) Date of filing: 31.10.85

(43) Date of publication of application:
13.05.87 Bulletin 87/20

(84) Designated Contracting States:
DE FR GB IT SE

(71) Applicant: TEXACO DEVELOPMENT CORPORATION
2000 Westchester Avenue
White Plains New York 10650(US)

(72) Inventor: Grigsby, Robert Allison, Jr.
216 Deepwood
Georgetown Texas 78626(US)
Inventor: Knifton, John Frederick
10900 Catskill Trail
Austin Texas 78750(US)

(74) Representative: Brock, Peter William et al
Michael Burnside & Partners 2 Serjeants' Inn
Fleet Street
London EC4Y 1HL(GB)

(54) **Process for preparing ethylene glycol from syngas.**

(57) The catalytic conversion of syngas (carbon monoxide and hydrogen) into mixtures of organic alcohols exhibits improved yields and improved selectivity to ethylene glycol when the catalyst comprises a ruthenium carbonyl compound, a rhodium carbonyl compound and a manganese carbonyl compound, all dispersed in a quaternary phosphonium compound, and dissolved in an N-alkyl-2-pyrrolidone solvent.

EP 0 221 214 A1

## PROCESS FOR PREPARING ETHYLENE GLYCOL FROM SYNGAS

This invention relates to a new process for preparing ethylene glycol and ethylene glycol derivatives from syngas using a novel catalyst system which allows for improved yields and selectivity to ethylene glycol.

More particularly, the invention provides a new and improved process for preparing improved yields of mixtures comprising ethylene glycol, ethylene glycol monomethyl ether, ethylene glycol monoethyl ether, methanol, and ethanol, by contacting a mixture of carbon monoxide and hydrogen with a catalyst comprising ruthenium, rhodium and manganese carbonyl compounds, all dispersed in a low melting quaternary phosphonium compound dissolved in an N-alkyl pyrrolidone solvent.

Ethylene glycol is a chemical which has found wide use in industry. It is used, for example, in the preparation of plasticizers for vinyl polymers and as a component in polyester fibres and antifreeze formulations. In view of its many uses, there is a need to find new and more economical methods for preparing it.

Proposed methods of making ethylene glycol include the reaction of carbon monoxide with hydrogen in the presence of various catalyst systems. In general, the mixture of carbon monoxide and hydrogen, commonly known as synthesis gas or syngas, is reacted at elevated temperatures and pressures in the presence of proposed catalysts. For example, BE-A-793086 and US-A-3940432, describe the cosynthesis of ethylene glycol and methanol using a complex rhodium catalyst. US-A-3833634 describes the use of various other metals as catalysts, but indicates that only rhodium and cobalt were effective in producing ethylene glycol, a typical yield of ethylene glycol being 9.8 parts out of 58 parts.

US-A-3989799 discloses a series of carbonyl mixed metal salts useful as catalysts in the reaction between carbon monoxide and hydrogen to produce oxygenated compounds. No selectivities are reported for particular products such as ethylene glycol, and there is no mention of the use of a quaternary "onium" salt.

US-A-4013700 discloses a process for producing polyhydric alcohols, and their ether and ester derivatives, in the presence of a quaternary phosphonium cation and a rhodium carbonyl complex. This system produces moderate quantities of ethylene glycol as demonstrated by yields of 4.2 grams and less.

US-A-4265828 discloses a process of making ethylene glycol which comprises heating a mixture of CO and $H_2$ with a catalyst system comprising a ruthenium compound dispersed in a low-melting quaternary phosphonium or ammonium base or salt under a pressure of 500 psi (3.5MPa) or greater at a temperature of at least 150°C. The highest yield of ethylene glycol obtained by this process was 17.6 weight %.

US-A-4315994 discloses the production of alkylene glycols and their ethers using a bimetallic catalyst system comprising ruthenium (III) acetylacetonate and rhodium (III) acetylacetonate dispersed in a low melting quaternary onium base or salt at a temperature of at least 150°C. The highest reported selectivity for ethylene glycol was 18.5 weight %.

Many of these processes are limited by the nature and activity of the catalyst systems. Most catalysts provide only moderate selectivity to the desired glycol, limited solubility, and/or are expensive to prepare.

It would be a significant advance in the art to provide an improved method for preparing ethylene glycol and monohydric alcohols, which provides ethylene glycol with greater yields and selectivity.

It has now been discovered that these and other desirable results may be accomplished by the process of the invention comprising contacting a mixture of carbon monoxide and hydrogen with a catalyst comprising a ruthenium carbonyl compound, a rhodium carbonyl compound and a manganese carbonyl compound dispersed in a low melting quaternary phosphonium salt dissolved in an N-alkyl pyrrolidone solvent at a temperature of 150 to 350°C and a pressure of 5 to 75 MPa. This process leads to the preparation of ethylene glycol and glycol derivatives including ethylene glycol monomethyl ether and ethylene glycol monoethyl ether, methanol and ethanol. It was surprising to find that by the use of this new catalyst system it is possible to obtain greater yields and selectivity in the formation of the ethylene glycol, which is formed in higher yields than were obtainable heretofore from syngas in related synthesis processes.

The process of the invention, as far as the formation of the desired ethylene glycol is concerned, may be represented by the following equation:

$$(1) \qquad 2\,CO + 3H_2 \qquad \begin{array}{c} CH_2OH \\ | \\ CH_2OH \end{array}$$

Typical yields of ethylene glycol, based on total liquid products, are from 10 to 40 wt%.

The ruthenium carbonyl compound used in the catalyst employed according to the invention may be a simple carbonyl compound such as triruthenium dodecacarbonyl, a hydrocarbonyl compound such as $H_2Ru_4(CO)_{13}$ or $H_4Ru_4(CO)_{12}$, or a substituted carbonyl compound such as the tricarbonyl-ruthenium (II) chloride dimer, $[Ru(CO)_3Cl_2]_2$. The preferred ruthenium compound is triruthenium dodecacarbonyl.

Suitable rhodium carbonyl compounds include simple carbonyls and hydrocarbonyls such as tetrarhodium dodecacarbonyl and hexarhodium hexadecacarbonyl, chlorodicarbonylrhodium(I) dimer and most preferably dicarbonyl (acetylacetonate)rhodium(I).

The manganese carbonyl compounds employed in the catalyst composition are preferably compounds having one manganese atom attached to three separate carbonyl groups and to an unsaturated hydrocarbon radical, e.g. compounds of the formula:

$Y\ Mn(CO)_3$

wherein Y is an unsaturated aliphatic or cycloaliphatic hydrocarbon group containing 2 to 16 carbon atoms, for example, allyl, cyclopentadienyl, cyclohexadienyl, and alkyl or aryl-substituted cycloaliphatic groups such as methylcyclopentadienyl, phenylcyclopentadienyl, or butylcyclohexadienyl. Examples of these include, among others, allyl manganese tricarbonyl, cyclohexadienyl manganese tricarbonyl, butadienyl manganese tricarbonyl, cyclohexenyl manganese tricarbonyl, methylcyclopentenyl manganese tricarbonyl.

Particularly preferred manganese carbonyl compounds include allyl manganese tricarbonyl, cyclopentadienyl manganese tricarbonyl and methylcyclopentadienyl manganese tricarbonyl.

The ruthenium, rhodium and manganese carbonyl compounds are first dispersed in a low melting quaternary phosphonium base or salt. The quaternary phosphonium base or salt selected must be relatively low melting, i.e. have a melting point below the temperature of the reaction. Usually quaternary phosphonium compounds employed have a melting point less than 180°C and preferably a melting point less than 150°C.

Suitable quaternary phosphonium salts have the formula:

$$\left[\begin{array}{ccc} & R_1 & \\ & | & \\ R_2 - & P & - R_3 \\ & | & \\ & R_4 & \end{array}\right]^+ X^-$$

wherein $R_1$, $R_2$, $R_3$ and $R_4$ are organic groups, particularly aliphatic hydrocarbon groups bonded to the phosphorus atom, and X is an anion, preferably chloride or bromide. The preferred organic groups include alkyl having 1 to 20 carbon atoms in a branched or linear chain, such as methyl, ethyl, n-butyl, iso-butyl, octyl, 2-ethylhexyl and dodecyl. Tetraethylphosphonium bromide and tetrabutylphosphonium bromide are typical examples presently in commercial production. The corresponding quaternary phosphonium acetates, benzoates and butyrates, are also satisfactory.

Illustrative examples of suitable quaternary phosphonium salts include tetrabutylphosphonium bromide, tetraheptylphosphonium bromide, tetrabutylphosphonium acetate, tetrabutylphosphonium benzoate, tetrabutylphosphonium butyrate, octylphosphonium acetate, tetrahexylphosphonium acetate and tetraoctylphosphonium bromide.

The preferred quaternary salts are generally tetralkylphosphonium salts containing alkyl groups having 1 to 20 carbon atoms, such as methyl, ethyl, butyl, amyl, hexyl and isobutyl, Tetrabutylphosphonium bromide is the most preferred.

The dispersion of the ruthenium carbonyl compound, rhodium carbonyl compound, and manganese carbonyl compound in the quaternary phosphonium compound is then dissolved in an N-alkyl-2-pyrrolidone solvent, such as N-(cyclohexyl)-2-pyrrolidone, N-(isopropyl)-2-pyrrolidone or N-methyl-2-pyrrolidone.

The quantity of ruthenium, rhodium and manganese carbonyl compounds employed in the process of the invention may vary over a wide range. The process is conducted in the presence of a catalytically effective quantity of each of the compounds which gives the desired product in a reasonable yield. The reaction proceeds when employing as little as 1 x $10^{-6}$ weight % or even less of the ruthenium and rhodium carbonyl compounds combined together, with as little as 1 x 10 $^{-6}$ weight % of the manganese carbonyl compound, or even less, based on the total weight of the reaction mixture. The upper concentration is dictated by a variety of factors, including catalyst cost, partial pressures of carbon monoxide and hydrogen and operating temperature. A concentration of from 1 x $10^{-6}$ to 20 weight % of total ruthenium carbonyl compound plus a rhodium carbonyl compound and preferably from 1 x $10^{-5}$ to weight %, and a manganese carbonyl compound concentration of from 1 x $10^{-6}$ to 20 weight %, preferably 1 x $10^{-5}$ to 10 weight %, based on the total weight of the reaction mixture, is generally desirable in the practice of this invention. The preferred ruthenium to rhodium to manganese atomic ratios are from 10:1:1 to 10:100:100. Most preferably, a relatively high ratio of ruthenium to rhodium is employed.

Particularly superior results are obtained when the above-noted five components of the catalyst system are combined as follows, on a molar basis: ruthenium carbonyl compound 0.1 to 4 mols, rhodium carbonyl compound, 0.1 to 4 mols; manganese carbonyl compound 0.1 to 4 mols, quaternary phosphonium compound 10 to 60 mols and solvent 0.1 to 60 mols.

The temperature which can be employed in the process of the invention may vary over a considerable range depending upon experimental factors, including the choice of catalyst, pressure and other variables. The range of operability is from 150 to 350°C. A narrower range of 150 to 275°C represents a particularly preferred temperature range.

The pressure employed may also vary over a considerable range, but in most instances is from 2.5 to 200 MPa. A preferred operating range varies from about 5 to 75 MPa, although pressures above 75 MPa also provide useful yields of the desired product. The pressures referred to herein represent the total pressure generated by all the reactants, although they are substantially due to the carbon monoxide and hydrogen fractions.

The relative amounts of carbon monoxide and hydrogen which can be initially present in the syngas mixture are variable, and these amounts may be varied over a wide range. In general, the mol ratio of $CO:H_2$ is from 20:1 to 1:20, and preferably from 5:1 to 1:5, although ratios outside these ranges may also be employed with good results. Particularly in continuous operations, but also in batch experiments, the carbon monoxide-hydrogen gaseous mixture may also be used in conjunction with up to 50% by volume of one or more other gases. These other gases may include one or more inert gases such as nitrogen, argon and neon, or they may include gases that may, or may not, undergo reaction under carbon monoxide hydrogenation conditions, such as carbon dioxide, hydrocarbons, such as methane, ethane, propane, and the like, ethers, such as dimethyl ether, methylethyl ether and diethyl ether, and alkanols, such as methanol.

The desired reaction product, ethylene glycol, will be formed in significant quantities, generally from 10 to 40 weight %. Also formed will be significant amounts of the lower monohydric alcohols, such as methanol and ethanol. Other derivatives, such as acetic acid and ethylene glycol ethers, may also be formed in minor amounts. The ethylene glycol, monohydric alcohols and other by-products can be recovered from the reaction mixture by conventional means, e.g. fractional distillation in vacuo.

The novel process of the invention can be conducted in a batch, semi-continuous or continuous manner. The catalyst can be initially introduced into the reaction zone batchwise, or it may be continuously or intermittently introduced into such a zone during the course of the synthesis reaction. Operating conditions can be adjusted to optimize the formation of the desired ethylene glycol product, which may be recovered by methods known to the art, such as distillation, fractionation or extraction. A fraction rich in the catalyst components may then be recycled to the reaction zone, if desired, and additional product generated.

The products have been identified in this work by one or more of the following analytical procedures: viz, gas-liquid phase chromatography (glc), gas chromatography/infrared spectroscopy (CG/IR), nuclear magnetic resonance (nmr) and elemental analyses, or a combination of these techniques. Analyses have, for the most part, been by parts by weight; percentages are by weight, all temperatures are in degrees Centigrade and all pressures in megaPascals.

To illustrate the process of the invention, the following examples are given.

EXAMPLE 1 (COMPARISON)

This Example omits a solvent. A mixture of triruthenium dodecacarbonyl (0.213g, 0.33 mmol), dicarbonyl(acetylacetonate) rhodium (I) (0.258g, 1.0 mmol), tetrabutylphosphonium bromide (5.0g, 14.7 mmol) and methyl cyclopentadienylmanganese tricarbonyl (0.055g, 0.25 mmol) was transferred to a glass-linear under $N_2$ purge to a 550-ml capacity pressure reactor equipped with heating and means of agitation. The reactor was sealed, flushed with $H_2$/CO (1:1) and pressurised to 20.8 MPa with $H_2$/CO (1:1). The reaction mixture was heated to 220°C with rocking, the pressure was raised to 36.7 MPa with $H_2$/CO (1:1) addition from the large surge tank and was maintained at 36.7 MPa by incremental additions from the surge tank. The reactor was held at temperature for 5 hours. hours.

On cooling, the excess gas was vented, the dark-red liquid product (10.4g) was recovered and analyzed.

Typical data for the distillate product on a solvent-free basis were as follows:
31.4% methanol
12.1% ethanol
3.6% ethylene glycol monomethyl ether
1.1% ethylene glycol monoethyl ether
29.2% ethylene glycol
Analysis of typical gas samples show the presence of:
48.8% hydrogen
2.1% carbon dioxide
0.3% methane
49.9% carbon monoxide
49.0% carbon monoxide
The liquid yield increase was 4.9g or 88.2%. The calculated yield of ethylene glycol was 23 mmols. The calculated total yield of ethylene glycol products was 26 mmols.

EXAMPLE 2

Example 2 was conducted in the same way as Example 1 but a solvent was included. A definite improvement in yields of ethylene glycol was observed. The reaction mixture contained triruthenium dodecacarbonyl (0.213g, 0.33 mmol), dicarbonylacetylacetonate rhodium (1) (0.258g, 1.0 mmol), tetrabutylphosphonium bromide (5.0g, 14.7 mmol) and methyl cyclopentadienylmanganese tricarbonyl (0.055g, 0.25 mmol) dissolved in 5.0g of 1-cyclohexyl-2-pyrrolidone. It was treated exactly as in Example 1.

On cooling, the excess gas was vented, the dark-red liquid product (16.0g) was recovered and analyzed.
34.3% methanol
10.4% ethanol
1.9% ethylene glycol monomethyl ether
0.3% ethylene glycol monethyl ether
33.7% ethylene glycol
Analysis of typical gas samples showed the presence of:
47.6% hydrogen
4.6% carbon dioxide
0.5% methane
46.7% carbon monoxide
The liquid yield increase was 5.5g or 99.5% (solvent-free basis). The calculated yield of ethylene glycol was 27 mmols. The calculated total yield of ethylene glycol products was 29 mmols.

Examples 3 and 4 demonstrate the effectiveness of having a larger mol ratio of ruthenium-to-rhodium catalyst precursor. Example 4 includes a manganese component while Example 3 does not.

Higher ruthenium-to-rhodium mol ratios lead to increased weight gains and an increase in yield of ethylene glycol products.

EXAMPLE 3 (COMPARISON)

The procedure used in Example 3 was the same as that used in Example 1, except a large amount of triruthenium dodecacarbonyl (0.852g, 1.33 mmol) and a smaller amount of dicarbonylacetylacetonate rhodium (I) (0.200g, 0.78 mmol) were employed and tetrabutylphosphonium bromide (10.0g, 29.4 mmol) was dissolved in 5.0g of 1-cyclohexyl-2-pyrrolidone.

On cooling, the excess gas was vented, the dark red liquid product (25.8g) is recovered and analyzed. Typical data for the distillate product on a solvent-free basis are as follows:
34.7% methanol
18.6% ethanol
5.4% ethylene glycol monomethyl ether
0.4% ethylene glycol monoethyl ether
21.7% ethylene glycol
Analysis of typical gas samples showed the presence of:
46.6% hydrogen
5.3% carbon dioxide
1.5% methane
45.1% carbon monoxide

The liquid yield increase was 9.7g or 88.2% (solvent free basis). The calculated yield of ethylene glycol was 29 mmols. The calculated total yield of ethylene glycol products was 35 mmols.

EXAMPLE 4

A mixture of triruthenium dodecacarbonyl (0.852g, 1.33 mmol), dicarbonylacetylacetonate rhodium (I) - (0.200g, 0.78 mmol), tetrabutylphosphonium bromide (10.0g, 29.4 mmol), and methyl cyclopentadienylmanganese tricarbonyl (0.218g, 1.0 mmol) was dissolved in 5.0g of 1-cyclohexyl-2-pyrrolidone and treated as in Examples 1 to 3.

On cooling, the excess gas was vented, the dark-red liquid product (27.2g) was recovered and analyzed.

Typical data for the distillate product on a solvent-free basis were as follows:
38.5% methanol
17.7% ethanol
4.6% ethylene glycol monomethyl ether
1.7% ethylene glycol monoethyl ether
19.5% ethylene glycol
Analysis of typical gas samples showed the presence of:
42.9% hydrogen
6.8% carbon dioxide
22% methane
47.2% carbon monoxide
The liquid yield increase was 10.9g or 97.0% (solvent-free basis). The calculated; yield of ethylene glycol was 34 mmols. The calculated total yield of ethylene glycol products was 43 mmols.

The following Table summarizes the results of the Examples.

| MeOH[1] | EtOH[1] | Ethylene gly-Glycol Mono-methyl ether[1] | Ethylene Glycol Mono-ethylether[1] | Ethylene glycol[1] | mmols of ethylene glycol | mmols of Ethylene glycol products | Wt% of Ethylene glycol products |
|---|---|---|---|---|---|---|---|
| 38.4 | 24.7 | 4.7 | 2.4 | 10.8 | 14 | 22 | 17.9 |
| 41.5 | 24.7 | 3.1 | 1.5 | 12.7 | 8 | 10 | 17.2 |
| 30.7 | 11.2 | 3.5 | 1.1 | 31.8 | 23 | 25 | 36.4 |
| 31.4 | 12.1 | 3.6 | 1.1 | 29.2 | 23 | 26 | 33.9 |
| 34.3 | 10.4 | 1.9 | 0.3 | 33.7 | 27 | 29 | 35.9 |
| 34.7 | 18.6 | 5.4 | 0.4 | 21.7 | 29 | 35 | 27.5 |
| 38.5 | 17.7 | 4.6 | 1.7 | 19.5 | 34 | 43 | 25.8 |

1. Values are in weight %.

## Claims

1. A process for the preparation of mixtures containing ethylene glycol by contacting a mixture of carbon monoxide and hydrogen with a ruthenium-containing catalyst at a temperature of 150 to 350°C and a pressure of 2.5 to 200 MPa, characterized in that the catalyst comprises a ruthenium carbonyl compound, a rhodium carbonyl compound and a manganese carbonyl compound dispersed in a low-melting quaternary phosphonium compound and dissolved in an N-alkyl pyrrolidone solvent.

2. A process according to claim 1 characterized in that the ruthenium carbonyl compound is triruthenium dodecacarbonyl.

3. A process according to claim 1 or 2 characterized in that the rhodium carbonyl compound is dicarbonyl (acetylacetonate) rhodium (1).

4. A process according to any of claims 1 to 3 characterized in that the manganese carbonyl compound is a compound of the formula

$Y\ Mn(CO)_3$

in which Y is an unsaturated aliphatic or cycloaliphatic hydrocarbon group having 2 to 16 carbon atoms.

5. A process according to any of claims 1 to 4 characterized in that the quaternary phosphonium compound is tetrabutyl phosphonium bromide.

6. A process according to any of claims 1 to 5 characterized in that the solvent is N-(cyclohexyl)-2-pyrrolidone, N-(isopropyl)-2-pyrrolidone or N-methyl-2-pyrrolidone.

7. A process according to any of claims 1 to 6 characterized in that the catalyst comprises from $1 \times 10^{-5}$ to 10 weight % of total ruthenium carbonyl compound plus rhodium carbonyl compound, and from $1 \times 10^{-5}$ to 10 weight % of manganese carbonyl compound, based on total reaction mixture.

8. A process according to any of claims 1 to 7 characterized in that the ruthenium to rhodium to manganese atomic ratio is from 10:1:1 to 10:100:100.

9. A process according to any of claims 1 to 8 characterized in that the catalyst comprises, on a molar basis:

0.1 to 4 mols of ruthenium carbonyl compound
0.1 to 4 mols of rhodium carbonyl compound
0.1 to 4 mols of manganese, carbonyl compound
10 to 60 mols of quaternary phosphonium compound and
0.1 to 60 mols of N-alkyl pyrrolidone solvent.

10. A process according to any of claims 1 to 9 characterized in that the temperature is from 150 to 275°C and the pressure is from 5 to 75 MPa.

European Patent Office

# EUROPEAN SEARCH REPORT

Application number

EP 85 30 7887

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl.4) |
| X | EP-A-0 085 191 (UNION CARBIDE)<br>* Page 17, lines 1-9; page 20, lines 13-19; page 29, lines 8-24; page 31, line 16 - page 32, line 14; page 34, lines 12-13; page 46, line 15 - page 47, line 4; page 47, line 19 - page 48, line 7; page 65, examples 1-4 *<br>--- | 1-3,5-7,10 | C 07 C 29/15<br>C 07 C 31/20 |
| X | EP-A-0 084 682 (UNION CARBIDE)<br>* Page 16, lines 1-7; page 19, lines 2-17; page 27, lines 16-26; page 29, line 19 - page 30, line 21; page 32, lines 12-13; page 44, line 14 - page 45, line 3; page 45, line 18 - page 46, line 10; page 56, examples 1-4 *<br>--- | 1-3,5-7,10 | TECHNICAL FIELDS SEARCHED (Int. Cl.4) |
| X | US-A-4 396 726 (L.H. SIMONS)<br>* Page 1, abstract; column 3, line 1 - column 4, line 21; column 4, lines 27-51; column 4, line 62 - column 5, line 11 *<br>----- | 1,2,4,5,7,10 | C 07 C 29/00<br>C 07 C 31/00 |

The present search report has been drawn up for all claims

| Place of search | Date of completion of the search | Examiner |
|---|---|---|
| THE HAGUE | 04-06-1986 | KINZINGER J.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82